Europäisches Patentamt

European Patent Office

Office européen des brevets

(19)

(11) Publication number: **0 225 066**

**A2**

(12) **EUROPEAN PATENT APPLICATION**

(21) Application number: 86308617.9

(22) Date of filing: 05.11.86

(51) Int. Cl.⁴: **C07K 7/06** , **C07K 7/08** , **A61K 37/02** , **A61K 39/21** , **G01N 33/569**

(30) Priority: 16.11.85 CS 8286/85

(43) Date of publication of application:
**10.06.87 Bulletin 87/24**

(84) Designated Contracting States:
**AT BE CH DE FR GB IT LI LU NL SE**

(71) Applicant: **SPOFA spojené podniky pro zdravotnickou vyrobu**
**No 11a Husinecká**
**Praha 3(CS)**

(72) Inventor: **Krchnák, Viktor**
**No. 6 Talafusova**
**Praha 4(CS)**
Inventor: **Krojidlo, Milan**
**No. 555/3 Strelnicl**
**Praha 8(CS)**
Inventor: **Mach, Otokar**
**No. 65 Naf Krocinkou**
**Praha 9(CS)**

(74) Representative: **Wotherspoon, Graham et al**
**FITZPATRICKS 4 West Regent Street**
**Glasgow G2 1RS Scotland(GB)**

(54) **Antigen determinant peptides and a process for the preparation thereof.**

(57) Antigen determinant peptides of the general formula I

H -Val -X -Y (I)

in which X represents an octapeptidic to undecapeptidic sequence of a defined structure
Tyr-Tyr-Arg-Asp-Ser-Arg-Asn-Pro-Leu,
Phe-Ile-His-Asn-Phe-Lys-Arg-Lys-Gly,
Val-Pro-Arg-Arg-Lys-Ala-Lys-Ile,
Leu-His-Thr-Gly-Glu-Arg-Asp-Trp-His-Leu-Gly,
Ser-Gly-Lys-Ala-Arg-Gly-Trp-Phe
or Ser-Ile-Glu-Trp-Arg-Lys-Lys-Arg-Tyr-Ser
and Y stands for a hydroxyl or an amino group,
available by fragment synthesis or successive condensation of the respective amino acid residues - (stepwise build-up) in solution or on a solid carrier, are expected to be of use as diagnostic agents for the so-called acquired immunodeficiency syndrome (AIDS), and also to have a potential as monodeterminant antibodies and safe synthetic vaccine agents combating the human T-cell lymphotropic type III virus (HTLV-III, HIV).

EP 0 225 066 A2

## Antigen Determinant Peptides and a Process for the Preparation thereof

The present invention relates to antigen determinant peptides of the general formula I

H -Val -X -Y (I)

in which X represents an octapeptidic to undecapeptidic sequence of the structure

Tyr-Tyr-Arg-Asp-Ser-Arg-Asn-Pro-Leu,

Phe-Ile-His-Asn-Phe-Lys-Arg-Lys-Gly,

Val-Pro-Arg-Arg-Lys-Ala-Lys-Ile,

Leu-His-Thr-Gly-Glu-Arg-Asp-Trp-His-Leu-Gly,

Ser-Gly-Lys-Ala-Arg-Gly-Trp-Phe

or Ser-Ile-Glu-Trp-Arg-Lys-Lys-Arg-Tyr-Ser

and Y stand for a hydroxyl or an amino group, and to process for their preparation.

The above defined peptide compounds are novel substances and proved to possess antigen determinant properties of the human T-cell lymphotropic type III virus (HTLV-III, HIV). They are expected to be of use as suitable diagnostic agents for the detection of antibodies active against the aforementioned virus eliciting the so-called acquired immunodeficiency syndrome (AIDS). They are also supposed to have a potential for obtaining monodeterminant antibodies and as future safe agents for the immunization against infections by the mentioned viral orginator of AIDS.

The following abbreviations are used hereinafter for convenience:

AIDS acquired immunodeficiency syndrome

ARC AIDS-related complex

HIV human immunodeficiency virus

HTLV human T-cell lymphotropic virus (same as HIV)

ELISA enzyme-linked immunosorbent assay

HPLC high-performance liquid chromatography

Rt retention time in minutes

Chemical agents and groups:

DCC N,N'-dicyclohexylcarbodiimide,

DCM dichloromethane, HOBt N-hydroxybenzotriazole,

TEA triethylamine, TFA trifluoroacetic acid,

TFMSA trifluoromethanesulfonic acid

Boc t-butyloxycarbonyl, Bzl benzyl, For formyl,

Tos p-toluenesulfonyl (tosyl),

Z benzyloxycarbonyl, BrZ o-bromobenzyloxycarbonyl

Amino acid residues:

Ala alanyl, Arg arginyl, Asp aspartyl,

Asn asparaginyl, Glu glutamyl, Gln glutaminyl,

Gly glycyl, His histidyl, Ile isoleucyl,

Leu leucyl, Lys lysyl, Phe phenylalanyl,

Pro prolyl, Ser seryl, Thr threonyl,

Trp tryptofyl, Tyr tyrosyl, Val valyl.

Heretofore, the immunodeficiency syndrome is recognized by the presence of the respective HIV-antagonizing antibodies in the blood serum of patients. This assay is effected with the use of the so-called ELISA (enzyme-linked immuno absorbent assay) test, which is based on an interaction of the patient's serum antibodies with the antigen, ie the human immunodeficiency virus inactivated by a tenside coating of the used ELISA test plates. The virus is only available by a rather complex multistage technique: the originator is first reproduced by infected host cells in a tissue culture. Then, the virus particles are isolated from the culture, medium and purified by several successive operations before the biological material can be used for the preparation of the ELISA test plate coating. The procedures involved are very laborious and expensive, and above all they are highly hazardous to operators since all this work is done with an extremely contagious pathogenic substrate. Even the ELISA test plates, though they are provided with an inactivated preparation, must be handled as a potentially infectious material. The above-outlined principle is adopted in manufacturing the laboratory AIDS diagnostic sets as supplied by several major medicopharmaceutical producers. The test is not completely specific: it delivers a certain minor percent of false positive responses that are due to unavoidable residual contaminants from the tissue culture. In view of the reported incidence of those non-negligible falsely positive responses, all positive findings must be verified by performing additional, confirmation tests based on another principle, either by the immunofluorescence assay or by the so-called Western blot method. These disadvantages can be eliminated or substantially diminished by the recent development of a novel assay set using new antigen determinant peptides of the present invention.

The subject compounds of the invention are available either by fragment synthesis or by successive condensation of the respective amino acid residues (stepwise build-up) in solution or on a polymeric solid carrier. A preferable process, which is described in detail in the present application, is based on the Merrifield solid-phase technique of peptide synthesis and substantially resides in successive steps comprising first attaching to reactive groups of a modified polystyrene-type carrier resin the appropriately protected carboxyterminal amino acid residue of the sequence, subsequent synthesis of the required peptide chain by a series of successive reaction cycles using the respective protected amino acids and conventional condensing agents, acidolytic cleavage of the obtained peptidyl resin, during which operation the formed peptide chain is detached from the carrier resin and simultaneously deprotected, and final purification of the resulting peptide product.

Thus, polystyrene crosslinked with divinylbenzene, preferably in an amount of I wt %, and modified by incorporation of chloromethyl or benzhydrylamine moieties was employed as the carrier. N$^{alpha}$ amino groups of the acids were protected by t-butyloxycarbonyl (Boc) groups (other acidically removable protective groups can also be used, but do not bring in additional advantages), and the acidolytic deprotection in the respective stages of the synthesis was conveniently effected using a solution of trifluoroacetic acid in dichloromethane. Side functional groups (as a rule hydroxyls or amino groups) of bi-and multi-functional amino acids were protected with benzyl or related (eg, benzyloxycarbonyl, Z) groups. The successive condensations of protected amino acids were effected with the use of suitable reactive derivatives of acids, advantageously with symmetric anhydrides or N-hydroxybenzotriazol (HOBt) esters. After completion of the synthesis, the formed peptide chains were split acidically off the resin under simultaneous removal of protective groups from the bi-and multi-functional amino acid residues. This cleavage was conveniently performed under the action of liquid hydrogen fluoride or trifluoromethanesulphonic acid in the presence of trifluoroacetic acid and thioanisole. The obtained crude products were purified either by column chromatography on carriers routinely employed in the preparative chemistry of peptides, preferably on silica gel provided with a hydrophobic aliphatic hydrocarbon coating, or on molecular sieve carriers. The composition of the resulting peptide products was confirmed by amino acid analysis, and their purity was verified by current chromatographic and electrophoretic separation methods.

The amino acid sequences of the peptides of formula I were derived as fragments of the amino acid sequences of the HIV (human immunodeficiency virus) proteins and selected so as to include the respective antigen determinants. The resulting peptides were found to be useful primarily for diagnostic purposes, more particularly for the diagnosing of HIV in body fluids and biological substrates such as blood serum by the detection of antibodies produced by certain cells of HIV-infected individuals and antagonizing the pathogenic HIV proteins. The diagnostic examination is based on an interaction of the subject antigen determinant peptide with HIV antibodies formed in the organism infected by the virus. The examination procedure can be advantageously performed on the principle of the standard enzyme-linked immunosorbent assay (ELISA) test, using a modified test set substantially in the following manner. The microwells provided in the test plates (non-coated, antigen-free plastic supports) are first coated with the antigen determinant peptide: they are filled with a solution of this peptide, or preferably its conjugate with a suitable macromolecular vehicle ensuring proper adhesion to the test plate support material - (polystyrene) and non-interfering in the assay, eg, caprine or bovine serum albumin, in a pH 7.0 phosphate or pH 9.5 carbonate buffer. After an incubation period of 4 hours at 20°C and 44 hours at 4°C, the supernatant solution containing the remaining free, nonsorbed peptide is decanted and reused for further coating operations. The plate wells are rinsed with phosphate buffer solution and, for the purpose of blocking of unoccupied receptory sites on the plastic surface, filled with a 3% solution of bovine serum albumin in the same phosphate buffer. After one hour of incubation at 37°C, the wells are rinsed with phosphate buffer and the patients' sera to be examined, previously diluted 1:100, are introduced. The test plates are incubated for 2 hours at 37°C, the wells are rinsed successively with phosphate buffer and water, and a conjugate of animal antibody to human immunoglobulin with horse-radish peroxidase is added. After 2 hours of incubation at 37°C, the wells are thoroughly washed to remove solubles, and the conjugated peroxidase is visualized by the standard immunologic colour reaction, eg, with 3,3', 5,5'-tetramethylbenzidine or o-phenylenediamine and hydrogen peroxide. The colour development may require a period of up to 30 minutes. The colour intensity is measured spectrophotometrically at the wavelength prescribed for the reagent used. Thus, in the above procedure, the absorbances are read at 450 or 492 nm respectively. The colour reaction using the subject peptides gave an average relative absorbance of 41.6% as compared to the positive control value taken as 100% obtained with an antigen prepared from native biological material (inactivated live HIV). When a negative, antigen-free control serum was used for comparison, the colour response fluctuated at the background level of the measurement.

The antigen determinant peptides of the present invention are also expected to find use for obtaining monodeterminant antibodies to pathogenic HIV proteins and for active immunization against HIV. After preliminary in vivo experiments on several species of experimental animals, the immunization can conveniently be effected using the subject peptides, or their mixtures either in the free state, or preferably attached to, or incorporated by means of bi-functional polycondensing agent into an appropriate carrier structure. Thus, the authors succeeded in immunizing animals, especially rabbits, with these peptides free or advantageously polycondensed with glutaraldehyde or attached to bovine serum albumin, thyreoglobulin or limpet keyhole hemocyanin, eg, using N,N'-dicyclohexylcarbodiimide. Freund complete adjuvans (with inactivated Koch bacilli) or muramyldipeptide was used as immunoadjuvans.

As shown by the reported experimental results, the subject peptides can be of use as diagnostics in screening for HIV and presumably also as safe vaccinating agents for active immunization of mammals against this viral originator of AIDS.

Further particulars of the preparative procedure are illustrated by the subsequent non-limitative examples.

Example I

H-Val-Tyr-Tyr-Arg-Asp-Ser-Arg-Asn-Pro-Leu-OH

A peptide synthesizer vessel was charged with 2.5 g of a chloromethylated polystyrene resin crosslinked with 1% of divinylbenzene and esterified with Boc-Leu (Leu content 0.6 mmole/g of resin). The synthesis included 9 reaction cycles each consisting of the following steps:

I) splitting off protective groups (deprotecting), TFA -DCM 1:1, 1 $\times$ 5 and 1 $\times$ 30 min,

2) washing, DCM, 3 $\times$ 1 min,

3) washing, 2-propanol, 2 $\times$ 1 min,

4) washing, DCM, 2 $\times$ 1 min,

5) neutralization, TEA -DCM 1:9, 1 $\times$ 1 min and 1 $\times$ 3 min,

6) washing, DCM, 6 $\times$ 1 min,

7) condensation, unless otherwise stated using preformed symmetric anhydrides (300% molar excess) until negative reaction for amino groups, and

8) washing, DCM, 3 $\times$ 1 min.

In the mentioned 9 reaction cycles, the following protected amino acids were successively attached:

Boc-Pro, Boc-Asn (condensation was effected using preformed HOBt esters, 300% molar excess), Boc-Arg(Tos) (by mixing this amino acid derivative with DCC as a condensing agent in the reaction vessel), Boc-Ser(Bzl), Boc-Asp(OBzl), Boc-Arg-(Tos) (similarly as above for this Arg derivative), Boc-Tyr(Z) twice and Boc-Val.

On completion of the synthesis 4.1 g of the peptidyl resin was obtained. A sample (0.5 g) of this material was suspended in a TFMSA (0.75 ml) -TFA (2.5 ml) -thioanisole (1 ml) mixture. After one hour of stirring at ambient temperature, the product was precipitated with anhydrous ether (100 ml), separated, washed with the same solvent, extracted into 1 M acetic acid and purified on a polyacrylamide gel column (1 $\times$ 100 cm) using 1 M

acetic acid (flow rate 7 ml/hour) as the mobile phase. Fractions containing a homogenous product were combined and freeze-dried. The resulting peptide material (yield 113 mg) had an amino acid analysis consistent with theoretical values for the title composition, and its purity was verified by analytical HPLC (Rt 7.9 in MeOH 40% - 0.1% aqueous TFA solution 60%), thin-layer chromatography and paper electrophoresis at pH 2.5. $R_f$ 0.45 (n-butanol -acetic acid -water, 4:1:1)

Analogous procedure using the appropriate amino acid derivatives afforded the following peptide compounds:

H-Val-Phe-Ile-His-Asn-Phe-Lys-Arg-Lys-Gly-OH - (yield 57 mg),

Rt 6.8 (MeOH) 45% -0.1% aqueous TFA solution 55%)

$R_f$ 0.15 (BuOH -AcOH - water, 4:1:1)

H-Val-Val-Pro-Arg-Arg-Lys-Ala-Lys-Ile-OH (yield 79 mg),

Rt 6.1 (MeOH 35% -0.1% aqueous TFA solution 65%)

$R_f$ 0.22 (same system as above)

Example ·

H-Val-Leu-His-Thr-Gly-Glu-Arg-Asp-Trp-His-Leu-Gly-NH$_2$

A peptide synthesizer vessel was charged with 1.5 g of a benzhydrylamine-containing polystyrene resin crosslinked with 1% of divinylbenzene (amino group content 0.7 mmole/g of resin). The synthesis included 12 reaction cycles each consisting of the same steps as in Example I with the only difference that the first cycle started with step 4), and condensations were effected using a 300% molar excess of the corresponding HOBt esters. The following protected amino acid residues were successively attached in the respective cycles:

Boc-Gly, Boc-Leu, Boc-His(Boc), Boc-Trp(For), Boc-Asp(OBzl), Boc-Arg(Tos) (condensed similarly as this amino acid derivative in Example I), Boc-Glu(OBzl), Boc-Gly, Boc-Thr(Bzl), Boc-His(Boc), Boc-Leu and Boc-Val.

On completion of the synthesis, 3.1 g of the peptidyl resin was obtained. A sample (0.5 g) of this material was cleaved for one hour at 0°C using 10 ml of a 10% solution of p-thiocresol in liquid HF. The formed peptide chain was so split off the resin and simultaneously deprotected. The resulting crude product was purified by preparative HPLC on a column (2 $\times$ 50 cm) using silica gel coated with hydrophobic aliphatic chains as the stationary phase and 0.1% TFA solution in 25% aqueous methanol, and uniform fractions were combined and freeze-dried. The pure product (yield 72 mg) had an amino acid analysis corresponding to theoretical values, and its purity was verified by analytical HPLC (Rt 8.2 in MeOH 40% -0.1% aqueous TFA solution 60%), thin-layer chromatography and paper electrophoresis at pH 2.5.

$R_f$ 0.40 (n-butanol -acetic acid -water, 4:1:1)

Analogous procedure using the appropriate amino acid derivatives gave the following peptide compounds:

H-Val-Ser-Gly-Lys-Ala-Arg-Gly-Trp-Phe-NH$_2$ (yield 72 mg),

Rt 7.1 (MeOH 40% -0.1% aqueous TFA solution 60%)

$R_f$ 0.50 (BuOH -AcOH -water, 4:1:1)

H-Val-Ser-Ile-Glu-Trp-Arg-Lys-Lys-Arg-Tyr-Ser-NH$_2$ (yield 46 mg),

Rt 5.7 (MeOH 35% -0.1% aqueous TFA solution 65%)

$R_f$ 0.47 (same system as above)


## Claims

1. Antigen determinant peptides of the general formula I

H -Val -X -Y (I)

in which X represents an octapeptidic to undecapeptidic residue of the structure

Tyr-Tyr-Arg-Asp-Ser-Arg-Asn-Pro-Leu,

Phe-Ile-His-Asn-Phe-Lys-Arg-Lys-Gly,

Val-Pro-Arg-Arg-Lys-Ala-Lys-Ile,

Leu-His-Thr-Gly-Glu-Arg-Asp-Trp-His-Leu-Gly,

Ser-Gly-Lys-Ala-Arg-Gly-Trp-Phe

or Ser-Ile-Glu-Trp-Arg-Lys-Lys-Arg-Tyr-Ser

and Y stands for a hydroxyl or an amino group.

2. The antigen determinant peptide of claim 1 having the formula

H-Val-Tyr-Tyr-Arg-Asp-Ser-Arg-Asn-Pro-Leu-OH.

3. The antigen determinant peptide of claim 1 having the formula H-Val-Leu-His-Thr-Gly-Glu-Arg-Asp-Trp-His-Leu-Gly-NH$_2$.

4. An immunoassay test for diagnosing the presence of HIV in a biological medium comprising contacting an antigen determinant peptide of the general formula I defined in claim 1 with said biological medium under conditions favourable to interaction of said peptide with HIV antibodies which may be present in the said medium, introducing a suitably labelled binding partner for said antibodies to provide a signal relating to the presence of said antibodies and monitoring the signal to determine the presence or absence of said antibodies.

5. An immunoassay test according to claim 4 wherein the antigen determinant peptide, or a conjugate thereof with a suitable non-interfering macromolecular vehicle, is coated on an initially antigen-free support having a multiplicity of receptor sites to obtain adsorption of the peptide to the support and said coated support is subsequently treated with a free-receptor site deactivating medium to avoid spurious interactions between the biological medium and the support.

6. An immunoassay test according to claim 4 or claim 5 wherein the labelled binding partner is a conjugate of an anti-immunoglobulin with a readily-detectable enzyme.

7. An immunoassay test according to claim 6 wherein the conjugate is one of animal anti-human immunoglobulin with horse-radish peroxidase, and detection is by means of spectrophotometry.

8. Use of an antigen determinant peptide of the general formula I defined in claim 1 for the production of monodeterminant antibodies to pathogenic HIV proteins by introducing said peptide to a viable cell system, and subsequently harvesting antibodies raised by said system.

9. A preparation for the immunization of a host against HIV comprising an antigen determinant peptide of the general formula I defined in claim 1, or a mixture thereof, either in the free state or combined with an appropriate carrier structure, and an immunoadjuvant.

10. A diagnostic kit for use in screening for HIV comprising an antigen-free support having a multiplicity of receptor sites on at least one surface thereof, an antigen determinant peptide of the general formula I defined in claim 1, optionally in the form of its conjugate with a suitable macromolecular vehicle to improve adsorption of said peptide to said support in use, a non-interfering receptor site blocking agent, and a determinable marker or labelling means having a specific affinity for HIV antibodies.


Claims for the following Contracting State : AT

1. A process for the preparation of antigen determinant peptides of the general formula I

H -Val -X -Y (I)

in which X represents an octapeptidic to undecapeptidic sequence of the structure

Tyr-Tyr-Arg-Asp-Ser-Arg-Asn-Pro-Leu,

Phe-Ile-His-Asn-Phe-Lys-Arg-Lys-Gly,

Val-Pro-Arg-Arg-Lys-Ala-Lys-Ile,

Leu-His-Thr-Gly-Glu-Arg-Asp-Trp-His-Leu-Gly,

Ser-Gly-Lys-Ala-Arg-Gly-Trp-Phe

or Ser-Ile-Glu-Trp-Arg-Lys-Lys-Arg-Tyr-Ser

and Y stands for a hydroxyl or an amino group, comprising attaching to chloromethyl or benzhydrylamine moieties of a polystyrene-type carrier resin the carboxyterminal amino acid residue of the sequence, subsequent successive synthesis of the required peptide chain using the respective protected amino acids and conventional condensing agents, acidolytic cleavage of the obtained peptidyl resin to liberate and deprotect the formed peptide, and final purification of the product.

2. A process according to claim 1 for the preparation of an antigen determinant peptide of the formula

H-Val-Tyr-Tyr-Arg-Asp-Ser-Arg-Asn-Pro-Leu-OH

comprising attaching to chloromethyl moieties of said carrier resin the Boc-protected carboxyterminal Leu residue, subsequent synthesis of the required peptide chain by successive condensation of Boc-Pro, Boc-Asn, Boc-Arg(Tos), Boc-Ser(Bzl), Boc-Asp(OBzl), Boc-Arg(Tos), Boc-Tyr(Z) twice and Boc-Val, cleavage of the peptidyl resin with trifluoromethanesulphonic acid and purification of the liberated peptide product by high-performance liquid chromatography.

3. A process according to claim 1 for the preparation of an antigen determinant peptide of the formula

H-Val-Leu-His-Thr-Gly-Glu-Arg-Asp-Trp-His-Leu-Gly-NH$_2$

comprising attaching to benzhydrylamine moieties of said carrier resin the Boc-protected carboxyterminal Gly residue, subsequent synthesis of the required peptide chain by successive condensation of Boc-Leu, Boc-His(Boc), Boc-Trp(For), Boc-Asp-(OBzl), Boc-Arg(Tos), Boc-Glu(OBzl), Boc-Gly, Boc-Thr(Bzl), Boc-His(Boc), Boc-Leu and Boc-Val, cleavage of the peptidyl resin with trifluoromethanesulphonic acid and purification of the liberated peptide product by high-performance liquid chromatography.

4. Antigen determinant peptides whenever prepared by a process substantially in accordance with any of the preceding claims or an obvious equivalent thereof.

5. An immunoassay test for diagnosing the presence of HIV in a biological medium comprising contacting an antigen determinant peptide of the general formula 1 defined in claim 1 with said biological medium under conditions favourable to interaction of said peptide with HIV antibodies which may be present in the said medium, introducing a suitably labelled binding partner for said antibodies to provide a signal relating to the presence of said antibodies and monitoring the signal to determine the presence or absence of said antibodies.

6. An immunoassay test according to claim 5 wherein the antigen determinant peptide, or a conjugate thereof with a suitable non-interfering macromolecular vehicle, is coated on an initially antigen-free support having a multiplicity of receptor sites to obtain adsorption of the peptide to the support and said coated support is subsequently treated with a free-receptor site deactivating medium to avoid spurious interactions between the biological medium and the support.

7. An immunoassay test according to claim 5 or claim 6 wherein the labelled binding partner is a conjugate of an anti-immunoglobulin with a readily-detectable enzyme.

8. An immunoassay test according to claim 7 wherein the conjugate is one of animal anti-human immunoglobulin with horse-radish peroxidase and detection is by means of spectrophotometry.

9. Use of an antigen determinant peptide of the general formula 1 defined in claim 1 for the production of monodeterminant antibodies to pathogenic HIV proteins by introducing said peptide to a viable cell system and subsequently harvesting antibodies raised by said system.

10. A diagnostic kit for use in screening for HIV comprising an antigen-free support having a multiplicity of receptor sites on at least one surface thereof, an antigen determinant peptide of the general formula 1 defined in claim 1, optionally in the form of its conjugate with a suitable macromolecular vehicle to improve adsorption of said peptide to said support in use, a non-interfering receptor site blocking agent, and a determinable marker or labelling means having a specific affinity for HIV antibodies.